(19) Europäisches Patentamt
European Patent Office
Office européen des brevets

(11) Publication number : **0 472 320 B1**

# (12) EUROPEAN PATENT SPECIFICATION

(45) Date of publication of patent specification : **12.01.94 Bulletin 94/02**

(51) Int. Cl.$^5$ : **A61K 7/50**

(21) Application number : **91307160.1**

(22) Date of filing : **05.08.91**

## (54) Acyl isethionate skin cleansing compositions containing selected betaines.

(30) Priority : **07.08.90 US 563468**

(43) Date of publication of application : **26.02.92 Bulletin 92/09**

(45) Publication of the grant of the patent : **12.01.94 Bulletin 94/02**

(84) Designated Contracting States : **CH DE ES FR GB IT LI NL SE**

(56) References cited :
**EP-A- 0 176 330**
**EP-A- 0 203 750**
**EP-A- 0 227 321**
**EP-B- 0 117 135**
**US-A- 4 137 191**

(73) Proprietor : **UNILEVER PLC**
**Unilever House Blackfriars P.O. Box 68**
**London EC4P 4BQ (GB)**
(84) **GB**
Proprietor : **UNILEVER N.V.**
**Weena 455**
**NL-3013 AL Rotterdam (NL)**
(84) **CH DE ES FR IT LI NL SE**

(72) Inventor : **Ashley, Jeanette Frances, née Carque**
**c/o Unilever Research US Inc. 45 River Road**
**Edgewater, NJ 07020 (US)**
Inventor : **Coxon, Andrew Charles**
**Unilever Res.Port Sunlight Lab. Quarry Road East**
**Bebington, Wirral Merseyside L63 3JW (GB)**
Inventor : **Lee, Robert Stanley**
**Unilever Res.Port Sunlight Lab. Quarry Road East**
**Bebington Wirral Merseyside L63 3JW (GB)**

(74) Representative : **Fransella, Mary Evelyn et al**
**Unilever PLC Patent Division Colworth House**
**Sharnbrook**
**Bedford MK44 1LQ (GB)**

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

EP 0 472 320 B1

Jouve, 18, rue Saint-Denis, 75001 PARIS

**Description**

## BACKGROUND OF THE INVENTION

## FIELD OF THE INVENTION

The invention relates to solid skin cleansing compositions containing major amounts of acyl isethionates and selected betaines. These compositions exhibit improved skin mildness.

## PRIOR ART

Traditionally, soap has been utilised as a skin cleanser. Soap is, however, a very harsh chemical. Irritated and cracked skin result from use of soap, especially in colder climates. There are, however, certain benefits from the use of soap including low cost, ease of manufacture into bars, and good lathering properties.

There has been much commercial activity in attempting to replace soaps with milder surfactants. The introduction of mild synthetic detergent toilet bars, especially those based on sodium cocoyl isethionates, has been particularly successful. Patents relating to this technology are fully discussed in U.S.Patent No.4954282, which is incorporated by reference herein.

Several publications describe acyl isethionate and betaines in a base composition. These compositions are normally intended to deliver some other ingredient.

In JP 62141098 acyl isethionate and carboxybetaine are included in a surfactant base for shampoos and soaps which contain alpha-glucan to impart a smooth feel to skin and hair. JP 60008398 discusses a similar utility in which dried marmelo fruit seed is used to impart a smooth feel to skin and hair.

An antidandruff shampoo with good cleaning and foaming composed of acyl isethionate/lauryl betaine mixtures is described in JP 60161498.

JP 60181200 discusses the use of a propane diol type antibiotic to prevent rancidity and improve perfume stability. Acyl isethionate and betaines are possible surfactants for the soap base.

In EP 0117135, acyl isethionate and carboxybetaines are included in a surfactant base for shampoos which contain polymers to improve anti-microbial and anti-dandruff efficacy.

JP 7614907 discusses the use of betaines in a hard transparent soap formulation. Acyl isethionate may also be included.

JP 63139998 discusses a skin detergent blended with clay mineral. Acyl isethionate and betaine are included as possible surfactants.

Amphoterics have also been cited in conjunction with other anionic surfactants to improve mildness. JP 59100199 describes mildness benefits when betaines are combined with nonionic and taurine coactives in cleaner compositions.

Use of cocamidopropyl betaine with diethanolamine lauryl sulfate in a shampoo composition which causes little eye irritation is described in U.S. patent No 4,137,191.

Liquid detergents with good skin compatibility containing betaine and nonylphenoxytri (ethyleneoxy) ethyl sulfate are described in DE 2017370.

U.S. patent No. 4,595,526 describes a primarily nonionic liquid composition with acyl isethionate and betaine which is mild to skin.

Several publications describe formulations containing betaines and fatty acid salts as liquid products (JP 63165499), bars (IL 69,378) and general body cleansers (JP 6104800).

The use of betaines with soap to provide good foaming and detergency and mildness to skin is described in JP 5131707 and JP 5131708.

U.S. 4,812,253 discusses various compositions containing polymeric skin mildness aids, moisturizers, soap and selected surfactants which include acyl isethionates and betaines.

The present invention provides compositions with excellent skin mildness while also maintaining good lather properties.

Thus, it is an object of this invention to provide a skin cleansing composition based upon acyl isethionates as the main actives in combination with betaines which are substantially milder to the skin than previously known compositions.

These and other objects of the invention will become more readily apparent through the following summary and detailed description.

## SUMMARY OF THE INVENTION

A solid skin cleansing composition is provided comprising:

i) selected acyl esters of isethionic acid salts in an amount of about 20 to 70% by weight;

ii) at least one betaine wherein the weight ratio of said acyl esters to betaine is about 10:1 to about 2:1.

iii) 0 to 3% by weight soap.

In order to provide a composition more acceptable to consumers, the following materials can additionally be used:

i) free fatty acid in an amount of about 2-40%;

ii) free isethionate in an amount of about 2-20%;

iii) soap in an amount of about 0-2%;

iv) water in an amount of about 2-20%;

v) miscellaneous in an amount of about 0-20%.

Ultra mild skin cleansing compositions, with excellent use properties are provided, based upon acyl esters of isethionate salts as the main active and a betaine, as a co-active. Preferred weight ratios of acyl isethionates to betaine are about 8:1 to 2:1, 10:1 to 5:1 and most preferred to maintain mildness are about 7:1 to 2:1.

## DETAILED DESCRIPTION OF THE INVENTION

In this invention, the irritancy of the main active, acyl isethionate, can be reduced significantly by the incorporation of selected betaines.

The cleansers resulting from this mixture of actives have superior skin mildness, excellent lather and good tactile characteristics. In addition, they are easily processable using standard manufacturing equipment.

Isethionate salts in the range of about $C_6$-$C_{18}$, such as those derived from coconut fatty acids, for example, have been employed in a number of commercial cleansing products and are known to produce a voluminous, creamy lather. This active, by itself, has been shown to be very mild to the skin. These actives are solid at room temperature and serve as excellent structurants when used in bar formulations.

The isethionate ester salts that may be employed herein are acyl ester isethionates and preferably the cocoyl ester. These preferred esters may be prepared from the usual cocoyl fatty acids having a small percentage of fatty acid chains below $C_8$ with over 95% of the carbon chain distribution being between $C_8$ and $C_{18}$ and more than half being $C_{12}$ or less. The acyl chain length of the esters will have at least about 90% $C_6$-$C_{18}$ and more than about 30% $C_{14}$ or lower. A typical cocoyl fraction will contain the chain length distribution in Table 1.

TABLE 1

| Chain Length | Wt. % Fatty Acid Combined As Cocoyl Isethionate |
|---|---|
| $C_{6-10}$ | 10-25 |
| $C_{12}$ | 45-55 |
| $C_{14-18}$ | 20-40 |
| $C_{18}$ unsaturated | 1-15 |

It is desirable for such isethionate based esters to be combined with selected betaines to provide mildness to the skin together with good bar properties such as good lather volume.

Betaines and amidobetaines are known to be very mild to both skin and eyes. The cocamidobetaines, in particular, have good lather properties.

Skin testing and use property evaluation have shown that the preferred ratio of isethionate to betaines is 2:1 to 8:1 or even as high as 10:1, preferably from about 2:1 to about 7:1, optimally about 4.5:1.

The betaines to be used in the invention may be any suitable betaine, either carboxybetaine or sulfobetaine with the following structure (I):

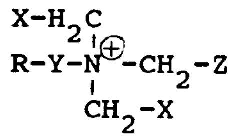

(I)

where R is any hydrocarbon chain having a distribution at least about 90% $C_5$-$C_{17}$; X is either a single hydrogen, H, or of the form $CH_2OH$; Y is either a methyl linkage, $CH_2$, or of the form $CONHCH_2CH_2CH_2$ (amidopropyl betaine); Z is either a carboxyl group, $COO^-$ (carboxybetaine), or of the form $CHOHCH_2SO_3$- (sulfobetaine or hydroxy sultaine); provided that when X, Y and Z are respectively H, $CH_2$ and $COO^-$ in combination, R is less than 50% $C_{15}$-$C_{17}$.

Preferably to ensure processability, mildness and other use properties the betaine is an amidopropyl betaine.

Specific betaines useful in the invention are lauryl betaine (Varion CDG from Sherex), cocamidopropyl betaine (Varion CADG from Sherex), coco betaine (Mackam CB from McIntyre), cocamidopropyl hydroxy sultaine (Varion CAS from Sherex) and tallow dihydroxyethyl glycinate (Varion TEG from Sherex).

The most preferable betaines are Cocoamidopropyl Betaine of the structure II:

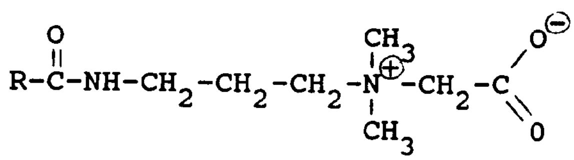

(II)

R in Structure II is derived from coconut fatty acids, but in other forms may be any convenient alkyl group. The chain length distribution of coconut fatty acids is similar to that for the coco group on the isethionate. The chain length distribution will contain at least 90% of $C_8$ to $C_{18}$ with more than half made up of $C_8$ to $C_{14}$. A typical fatty acid distribution in the cocoyl portion is the same as in Table 1.

As previously noted, soap may be somewhat harsh and when present in the compositions of this invention should be at a level no higher than about 35%, preferably less than 5%, and advantageously totally absent.

Free fatty acids of about 8-22 carbon atoms are desirably incorporated within the compositions of the present invention. Some of these fatty acids are present to operate as superfatting agents and others as skin feel and creaminess enhancers. Fatty alcohols, fatty amides and the like may also be employed. Superfatting agents enhance lathering properties and may be selected from fatty acids of carbon atoms numbering 8-18, preferably 10-18, in an amount up to 40% by weight of the composition. Skin feel and creaminess enhancers, the most important of which is stearic acid, are also desirably present in these compositions.

Other performance chemicals and adjuncts may be needed or employed with these compositions. The amount of these chemicals and adjuncts may range from about 0% to about 20% by weight of the total compositions. For instance, there may be included humectants such as glycerine; anti wear agents such as polymer JR and natural and synthetic gums and the like; germicides, perfumes, colourants, dyes, pigments such as titanium dioxide, electrolytes and water.

Evaluation of an active's skin mildness properties are determined through the following test procedure.

The Flex Wash Test

Each of the products' skin mildness properties were evaluated in a paired comparison with a control product using a standardised Flex Wash test. The Flex Wash test procedure consists of three daily two minute washes of the antecubital fossa (flex area of elbow). This method is an "exaggerated use" method designed to differentiate very mild products. Erythemal response varies only slightly with temperature and humidity fluctuations making the protocol suitable for year round testing.

Approximately 15 panellists were used as the test population. Panellist flex areas must be free of any skin condition (eczema, dryness, irritation, cuts or abrasions). Anyone taking antihistamines, anti-inflammatory drugs (more than 8 per week) or topical, oral or injectable cortisone or a regular basis was excluded from the study. The panel was divided into two sub-groups which were balanced for left handedness. Group I was assigned composition "A" for the left flex and "B" for the right flex. Group II reversed the order.

Following an evaluation, the panellist was instructed to moisten the left flex area, the sponge and test compositions formulated as toilet bars were dampened with tap water (100 ppm calcium/magnesium ions). The

sponge was then stroked over the test bar 10 times by the evaluator. The "dosed" sponge was placed in the panellist's right hand. The panellist then washed the left flex area for exactly two minutes. Thereupon, the flex was rinsed and patted dry. This washing procedure was repeated on the right arm with appropriate composition. Thus, both arms are tested simultaneously. Washing by this procedure was repeated three times daily for 5 consecutive days for a total of 15 washes. Treatment times were scheduled 1.5 hours apart. Each test site was evaluated immediately prior to washing and 4 hours after the third daily wash. A slightly different procedure was used for Example 7. In this evaluation, the flex area was only washed for one minute, repeated four times daily for five consecutive days for a total of 20 washes. The test site was evaluated immediately prior to each wash.

One trained assessor evaluated test sites for a total of 20 evaluations. The grading scale was as follows:

| | |
|---|---|
| 0 | - no erythema |
| 0.5 | - barely perceptible erythema |
| 1 | - mild spotty erythema/no edema |
| 1.5 | - mild/moderate erythema/with or without edema |
| 2 | - moderate confluent erythema/with or without edema or vesiculation |

Each test site was treated in the prescribed method until a grading of "2" or greater was attained or 15 washings had been completed. When a score of "2" or greater was attained, the treatment was discontinued on that flex. The final score was then carried through for all remaining evaluations. The remaining flex was washed until either a grading of at least "2" or 15 treatments were attained, whichever was first. In the Examples of this specification, the final grading, Mean Rank Scores, is the sum total of grade scores for 15 assessments per panellist averaged over the scores from all panellists. Thus, theoretically, the average score could range from 0 to 30; the lower score indicating absolutely no skin irritation while the 30 score being the most severe. Mean Endpoint Erythema scores are the mean of the evaluation scores, for each panellist, at which the first arm received a grade of "2" or greater erythema score or at the completion of fifteen washes. The Mean Rank Erythema scores are analysed and compared using the Wilcoxon Signed Rank Test (two-sample).

The products' skin mildness properties were evaluated by comparison to one of the following control formulations:

**Control Formulations**

| Component | % in Formulation | | |
|---|---|---|---|
| | 1 | 2 | 3 |
| Na Cocoyl Isethionate | 68.3 | 49.8 | 15.0 |
| Free Fatty Acid | 19.2 | 23.3 | 36.0 |
| Soap | - | 8.3 | - |
| Na Alkylbenzene Sulfonate | - | 2.0 | - |
| Myristamide Sulfosuccinate | - | - | 22.0 |
| Paraffin | - | - | 5.0 |
| Polyethylene Glycol 75 | - | - | 2.0 |
| Stearyl Alcohol | - | - | 2.0 |
| Myristamide | - | - | 4.0 |
| Na Isethionate | 6.5 | 4.7 | 1.5 |
| Miscellaneous | 1.0 | 6.6 | 4.0 |
| Water | 5.0 | 5.3 | 8.5 |

Control formulation 1 is a similar formulation to that of the test product with omission of the amphoteric coactive. Control formulation 2 is a formulation which is at parity with Control 1. Control formulation 3 is a product that is significantly milder than Control 2. The mildness criteria for this invention is that the test product is significantly milder than Controls 1 and 3 (p-value <0.07) or at parity with Control 3 (p-value >0.07).

The following examples will more fully illustrate the embodiments of this invention. All parts, percentages and proportions referred to herein and in the appended claims are by weight of the total composition unless

otherwise stated.

**EXAMPLES 1-3**

In examples 1-3, the acyl isethionate/betaine coactive ratio was varied from 6.5:1 to 2.9:1 to illustrate the relationship between surfactant ratio and mildness. A lauryl betaine coactive is used in these examples. Improved mildness is demonstrated by the Flex Wash results.

| Component | Formulation | | |
|---|---|---|---|
| | 1 | 2 | 3 |
| Na Cocoyl Isethionate | 60.1 | 57.8 | 53.7 |
| Lauryl Betaine | 9.3 | 13.4 | 18.7 |
| Stearic Acid | 13.3 | 12.8 | 11.9 |
| Coco Fatty Acid | 3.6 | 3.5 | 3.2 |
| Na Isethionate | 5.7 | 5.5 | 5.0 |
| Sodium Chloride | 0.6 | 0.9 | 1.2 |
| Miscellaneous | 0.9 | 0.8 | 0.8 |
| Water | 6.5 | 5.3 | 5.5 |
| cocoyl isethionate: betaine ratio | 6.5 | 4.3 | 2.9 |

**FLEX WASH**

| | | Mean Endpoint Erythema | | Mean Rank Score | | |
|---|---|---|---|---|---|---|
| Example | Control | Example | Control | Example | Control | p-value |
| 1 | 3 | 1.6 | 1.3 | 20.5 | 14.5 | 0.068 |
| 2 | 1 | 1.3 | 1.9 | 12.3 | 24.7 | 0.0002 |
| 3 | 1 | 0.8 | 2.0 | 10.7 | 26.3 | 0.0000 |

**EXAMPLES 4-7**

Superior mildness is demonstrated for the following cocoyl isethionate/betaine examples, where the betaine is coco betaine (Example 4), cocamidopropyl betaine (Example 5), cocamidopropyl hydroxy sultaine (Example 6) and tallow dihydroxyethyl glycinate (Example 7). The cocoyl isethionate to betaine ratio is about 4:1 for examples 4-6 and 7.8:1 for example 7.

| Component | Formulation | | | |
|---|---|---|---|---|
| | 4 | 5 | 6 | 7 |
| Na Cocoyl Isethionate | 56.1 | 56.8 | 56.9 | 60.6 |
| Coco Betaine | 13.9 | - | - | - |
| Cocamidopropyl Betaine | - | 13.2 | - | - |
| Cocamidopropyl Hydroxy-Sultaine | - | - | 13.7 | - |
| Tallow Dihydroxyethyl-Glycinate | - | - | - | 7.8 |
| Stearic Acid | 12.4 | 12.6 | 12.6 | 13.4 |
| Coco Fatty Acid | 3.4 | 3.4 | 3.4 | 3.7 |
| Na Isethionate | 5.3 | 5.4 | 5.4 | 5.7 |
| Sodium Chloride | 1.7 | 2.1 | 2.1 | 1.2 |
| Titanium Dioxide | - | - | - | - |
| Perfume | - | - | - | - |
| Miscellaneous | 0.8 | 0.8 | 0.8 | 0.9 |
| Water | 6.4 | 5.7 | 5.1 | 6.7 |

**FLEX WASH**

| | | Mean Endpoint Erythema | | Mean Rank Score | | |
|---|---|---|---|---|---|---|
| Example | Control | Example | Control | Example | Control | p-value |
| 4 | 3 | 1.2 | 1.2 | 17.5 | 17.5 | 1.000 |
| 5 | 3 | 1.1 | 1.3 | 16.1 | 18.9 | 0.396 |
| 6 | 3 | 1.2 | 1.0 | 21.6 | 17.4 | 0.232 |
| 7 | 1 | 1.2 | 1.5 | 10.7 | 16.3 | 0.056 |

## EXAMPLES 8-10

Examples 8-10 illustrate superior mildness for a cocoyl isethionate/cocamidopropyl betaine ratio of 6.6:1. Additional fatty acid is included in all three examples. Example 9 and 10 also include other additives, such as PEG-150 (Union Carbide Carbowax 8000) and dextrin (National Starch Nadex 360).

| Component | Formulation | | |
|---|---|---|---|
| | 8 | 9 | 10 |
| Na Cocoyl Isethionate | 49.8 | 38.9 | 43.5 |
| Cocamidopropyl Betaine | 7.6 | 5.9 | 6.6 |
| Stearic Acid | 23.7 | 23.5 | 23.5 |
| Coco Fatty Acid | 3.0 | 2.3 | 3.7 |
| Dextrin | - | 10.0 | 5.0 |
| Polyethylene Glycol 150 | - | 3.0 | 1.0 |
| Na Isethionate | 8.8 | 8.7 | 8.7 |
| Sodium Chloride | 1.2 | 1.0 | 1.1 |
| Titanium Dioxide | 0.5 | 0.5 | 0.5 |
| Perfume | 1.0 | 1.0 | 1.0 |
| Miscellaneous | 0.7 | 0.6 | 0.6 |
| Water | 3.7 | 4.6 | 4.8 |

**FLEX WASH**

| | | Mean Endpoint Erythema | | Mean Rank Score | | |
|---|---|---|---|---|---|---|
| Example | Control | Example | Control | Example | Control | p-value |
| 8 | 3 | 1.6 | 1.4 | 19.9 | 17.1 | 0.401 |
| 9 | 2 | 0.7 | 2.0 | 9.1 | 25.9 | 0.0000 |
| 10 | 3 | 1.1 | 1.2 | 14.9 | 18.1 | 0.3407 |

**EXAMPLE 11**

Lather volume measurements were performed on the forementioned formulations. A brief description of the lather and mush tests are reported below.

Objective Lather Volume - This test involves a trained assessor rotating the toilet bar 10 half turns from one face to the other face under running 35°C water to level out surface peculiarities and wet the bar. The water used is tap water of 70 to 80 ppm hardness as $CaCO_3$. 15 additional half turn rotations are performed immediately after removing the bar from the running water. The bar is then set aside and the lather on the hands is worked for 10 hand rotations. A measuring funnel is then inverted and placed over the hands and the hands and funnel are lowered together into a sink filled with room temperature distilled water. The measuring funnel has a cylindrical stem attached which is graduated in millilitre increments (usually from 0 to 125 ml). When the hands are fully immersed and the lather removed from the hands by the water, the hands are removed from beneath the funnel. The funnel is then lowered over the lather until the water reaches the 0 mark on the scale and the lather volume is then measured to the nearest 5 ml.

Example 11 illustrates the lather volume generated by each formulation of this invention. Each product produces a lather volume of 85ml or better. This value corresponds to a commercially acceptable level (e.g. Shield).

| Formulation | Acyl Isethionate: Betaine Ratio | Lather Volume (ml) |
|---|---|---|
| 1 | 6.5:1 | 111 |
| 2 | 4.3:1 | 98 |
| 3 | 2.9:1 | 92 |
| 4 | 4.0:1 | 85 |
| 5 | 4.3:1 | 86 |
| 6 | 4.2:1 | 95 |
| 7 | 7.8:1 | 87 |
| 8 | 6.6:1 | 101 |
| 9 | 6.6:1 | 114 |
| 10 | 6.6:1 | 99 |
| Dove | - | 115 |
| Shield | - | 85 |

The foregoing description and examples illustrate selected embodiments of the present invention.

**Claims**

1. A solid cleaning composition comprising:
   1) from about 20% to about 70% by weight acyl esters of isethionic acid salts, the acyl chain length distribution of said esters being at least about 90% $C_6$-$C_{18}$ and having more than about 30% $C_{14}$ or lower; and
   ii) at least one betaine wherein the weight ratio of said acyl esters to betaine is 10:1 to 2:1; and said betaine has the structure:

   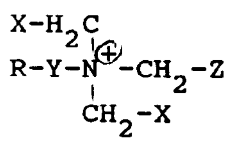

   wherein R is a hydrocarbon chain having a distribution of at least about 90% $C_5$-$C_{17}$;
   X is H, or $CH_2OH$;
   Y is $CH_2$ or $CONHCH_2CH_2CH_2$;
   Z is $COO^-$, or $CHOHCH_2SO_3^-$, provided that when X, Y and Z are respectively H, $CH_2$ and $COO^-$ in combination, R is less than 50% $C_{15}$-$C_{17}$; and
   iii) 0 to 3% by weight soap.

2. A composition according to claim 1 wherein the ratio of isethionate ester to betaine is from 8:1 to 2:1.

3. A composition according to claim 1 wherein the ratio of isethionate ester to betaine is from 10:1 to 5:1

4. A composition according to claim 1 wherein the ratio of isethionate ester to betaine is from 8:1 to 5:1.

5. A composition according to any preceding claim wherein the length of said acyl chain has the following distribution:

| Chain Length | % |
|---|---|
| $C_{6-10}$ | 10-25 |
| $C_{12}$ | 45-55 |
| $C_{14-18}$ | 20-40 |
| $C_{18}$ unsaturated | 1-15 |

**6.** A skin cleansing toilet bar composition comprising:
i) acyl esters of isethionic acid salts as defined in claim 1 in an amount of 20% to 70% by weight;
ii) at least one betaine as defined in claim 1 wherein the weight ratio of said acyl esters to betaine is 10:1 to 2:1;
iii) free fatty acid in an amount of 2-40% by weight;
iv) free isethionate in an amount of 2-20% by weight;
v) soap in an amount of 0-2% by weight;
vi) water in an amount of 2-20% by weight;
vii) miscellaneous in an amount of 0-20% by weight;

**7.** A composition according to claim 6 wherein the betaine is a carboxybetaine.

**8.** A composition according to claim 6 wherein the betaine is a sulfobetaine or hydroxy sultaine.

**9.** A composition according to claim 6 wherein the betaine is an amido propyl betaine.

**10.** A composition according to claim 9 wherein the betaine is a cocamidopropyl betaine.

## Patentansprüche

**1.** Feste Reinigungs-Zusammensetzung, umfassend:
i) etwa 20 bis etwa 70 Gew.-% Acylester von Isethionsäuresalzen, wobei die Verteilung der Acylkettenlängen dieser Ester so ist, daß mindestens etwa 90 % $C_6$-$C_{18}$ sind und mehr als etwa 30 % $C_{14}$ oder weniger sind; und
ii) mindestens ein Betain, wobei das Gewichtsverhältnis von Acylestern zu Betain 10:1 bis 2:1 ist; und wobei das Betain die Struktur:

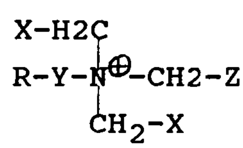

hat, worin R eine Kohlenwasserstoffkette ist mit einer solchen Verteilung, daß mindestens etwa 90 % $C_5$-$C_{17}$ sind;
X H oder $CH_2OH$ ist;
Y $CH_2$ oder $CONHCH_2CH_2CH_2$ ist;
Z $COO^-$ oder $CHOHCH_2SO_3^-$ ist, mit dem Vorbehalt, daß dann, wenn X, Y und Z H, $CH_2$ bzw. $COO^-$ in Kombination sind, weniger als 50 % der Reste R $C_{15}$-$C_{17}$ sind und
iii) 0 bis 3 Gew.-% Seife.

**2.** Zusammensetzung nach Anspruch 1, worin das Verhältnis von Isethionatester zu Betain 8:1 bis 2:1 ist.

**3.** Zusammensetzung nach Anspruch 1, worin das Verhältnis von Isethionatester zu Betain 10:1 bis 5:1 ist.

**4.** Zusammensetzung nach Anspruch 1, worin das Verhältnis von Isethionatester zu Betain 8:1 bis 5:1 ist.

**5.** Zusammensetzung nach einem der vorhergehenden Ansprüche, worin die Acylkettenlängen die folgende Verteilung haben:

| Kettenlänge | % |
|---|---|
| $C_{6-10}$ | 10-25 |
| $C_{12}$ | 45-55 |
| $C_{14-18}$ | 20-40 |
| $C_{18}$ ungesättigt | 1-15 |

6. Hautreinigungs-Seifenstück-Zusammensetzung, umfassend:
   i) Acylester von Isethionsäuresalzen, wie in Anspruch 1 definiert, in einer Menge von 20 bis 70 Gew.-%;
   ii) mindestens ein Betain, wie in Anspruch 1 definiert, wobei das Gewichtsverhältnis von Acylestern zu Betain 10:1 bis 2:1 ist;
   iii) freie Fettsäure in einer Menge von 2 bis 40 Gew.-%;
   iv) freies Isethionat in einer Menge von 2 bis 20 Gew.-%;
   v) Seife in einer Menge von 0 bis 2 Gew.-%;
   vi) Wasser in einer Menge von 2 bis 20 Gew.-%;
   vii) verschiedene Inhaltsstoffe in einer Menge von 0 bis 20 Gew.-%.

7. Zusammensetzung nach Anspruch 6, worin das Betain ein Carboxybetain ist.

8. Zusammensetzung nach Anspruch 6, worin das Betain ein Sulfobetain oder ein Hydroxysultain ist.

9. Zusammensetzung nach Anspruch 6, worin das Betain ein Amidopropylbetain ist.

10. Zusammensetzung nach Anspruch 9, worin das Betain ein Kokosamidopropylbetain ist.

## Revendications

1. Composition de nettoyage comprenant:
   i) d'environ 20% à environ 70% en poids d'esters acyliques de sels de l'acide iséthionique, la distribution de longueur de chaîne acyle desdits esters étant d'au moins 90% en $C_6$ à $C_{18}$ et possédant plus d'environ 30% en $C_{14}$ ou inférieur; et
   ii) au moins une bétaïne dans laquelle le rapport pondéral desdits esters acyliques à la bétaïne est de 10:1 à 2:1; et ladite bétaïne possède la structure:

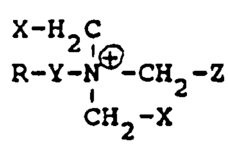

   dans laquelle R est une chaîne hydrocarbonée ayant une distribution d'au moins 90% en $C_5$ à $C_{17}$;
   X représente un atome d'hydrogène, ou $CH_2OH$;
   Y représente $CH_2$ ou $CONHCH_2CH_2CH_2$;
   Z représente $COO^-$, ou $CHOHCH_2SO_3^-$, pourvu que lorsque X, Y et Z représentent H, $CH_2$ et $COO^-$ en combinaison, R représente moins de 50% en $C_{15}$ à $C_{17}$; et
   iii) 0 à 3% en poids de savon.

2. Composition selon la revendication 1, dans laquelle le rapport d'ester d'iséthionate à la bétaïne est de 8:1 à 2:1.

3. Composition selon la revendication 1, dans laquelle le rapport d'ester d'iséthionate à la bétaïne est de 10:1 à 5:1.

4. Composition selon la revendication 1, dans laquelle le rapport d'ester d'iséthionate à la bétaïne est de 8:1 à 5:1.

5. Composition selon l'une quelconque des revendications précédentes, dans laquelle la longueur de ladite chaîne acyle possède la distribution suivante

| Longueur de chaîne | % |
|---|---|
| $C_6$ à $C_{10}$ | 10 à 25 |
| $C_{12}$ | 45 à 55 |
| $C_{14}$ à $C_{18}$ | 20 à 40 |
| $C_{18}$ insaturé | 1 à 15 |

6. Composition de pain de savon de nettoyage de la peau comprenant:
   i) des esters acyliques de sels de l'acide iséthionique tels que définis dans la revendication 1 en une quantité de 20% à 70% en poids;
   ii) au moins une bétaïne tel que défini dans la revendication 1 dans laquelle le rapport pondéral desdits esters acyliques à la bétaïne est 10:1 à 2:1;
   iii) un acide gras libre en une quantité de 2 à 40% en poids,
   iv) de l'iséthionate libre en une quantité de 2 à 20% en poids;
   v) un savon en une quantité de 0 à 2%;
   vi) de l'eau en une quantité de 2 à 20% en poids;
   vii) composants divers en une quantité de 0 à 20 pourcent en poids.

7. Composition selon la revendication 6, dans laquelle la bétaïne est une carboxybétaïne.

8. Composition selon la revendication 6, dans laquelle la bétaïne est une sulfobétaïne ou une hydroxybétaïne.

9. Composition selon la revendication 6, dans laquelle la bétaïne est une amidopropylbétaïne.

10. Composition selon la revendication 9, dans laquelle la bétaine est une cocamidopropyl bétaïne.